# FASCICULE DE BREVET EUROPEEN

(11) **EP 1 056 805 B1**
(45) Date de publication et mention de la délivrance du brevet: **24.03.2004**
(21) Numéro de dépôt: 99904906.7
(22) Date de dépôt: 17.02.1999
(51) Int. Cl.: C08L 33/24, C08L 25/18, A61K 7/00, A61K 7/06, A61K 7/48

(54) **POLYMERE EPAISSISSANT, PROCEDE DE PREPARATION ET APPLICATIONS EN COSMETIQUE**
POLYMERES VERDICKUNGSMITTEL, VERFAHREN ZU SEINER HERSTELLUNG UND VERWENDUNG IN KOSMETISCHEN ZUBEREITUNGEN
THICKENING POLYMER, PREPARATION METHOD AND USES IN COSMETICS

(30) Priorité: 17.02.1998 FR 9801918
(43) Date de publication de la demande: 06.12.2000
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: MALLO, Paul, F-78400 Chatou (FR); TABACCHI, Guy, F-81100 Castres (FR)
(74) Mandataire: Conan, Philippe Claude
(86) Numéro de dépôt international: PCT/FR1999/000353
(87) Numéro de publication internationale: WO 1999/042521

(56) Documents cités:
- EP-A- 0 503 853
- EP-A- 0 642 781
- EP-A- 0 815 846
- EP-B- 0 186 361

## Description

La présente demande concerne des Latex eau dans huile épaississants, leur procédé de préparation et leur application en tant qu'épaississant et/ou émulsionnant pour des produits de soins de la peau et des cheveux ou pour la fabrication de préparations cosmétiques, dermo-pharmaceutiques ou pharmaceutiques.

Différents épaississants existent et sont déjà utilisés pour ces usages. On connaît en particulier les produits naturels tels que les gommes de guar ou l'amidon mais dont les inconvénients sont ceux inhérents aux produits naturels, tels que la fluctuation des cours, les difficultés d'approvisionnement et une qualité aléatoire.

Les polymères synthétiques sous forme de poudre, principalement les polyacide-acryliques sont également largement utilisés mais présentent l'inconvénient de nécessiter une neutralisation lors de l'utilisation, car ils ne développent leur viscosité qu'à partir d'un pH > 6.5 et leur mise en solution est souvent fastidieuse.

Il existe aussi des polymères épaississants synthétiques, se présentant sous forme de latex inverse, c'est-à-dire dont la phase continue est une huile. La mise en solution de ces latex est extrêmement rapide ; les polymères contenus dans ces latex inverses, sont le plus souvent des copolymères acrylamide / acrylate de métal alcalin ou acrylamide/acrylamido 2-méthyl 2-propane sulfonate de sodium ; ils sont déjà neutralisés et lorsqu'ils sont mis en solution dans l'eau, par exemple à une concentration de 1%, on observe que le pH est généralement supérieur à 6.

Les copolymères acrylamide/acrylate de sodium ne développent cependant pas de propriétés épaississantes importantes lorsqu'on abaisse le pH en dessous de 6 ; par contre les copolymères acrylamide/acrylamido 2-méthyl 2-propanesulfonate de sodium décrits dans EP 0 503 853, gardent une capacité épaississante importante même à pH 4.

Cependant, de tels copolymères présentent des teneurs en monoacrylamide qui, bien qu'extrêmement faibles, pourraient conduire à rendre leur utilisation en cosmétique impossible dans un futur proche, suite à l'évolution de la législation européenne sur les substances dangereuses.

La demanderesse s'est donc intéressée à la synthèse et à la mise au point de polymères épaississants, même en pH acide, sous forme de latex inverse sans utiliser de mono-acrylamide.

L'invention a pour objet un procédé de préparation d'une composition comprenant une phase huile, une phase aqueuse, au moins un agent émulsifiant de type eau dans huile (E/H), au moins un agent émulsifiant de type huile dans eau (H/E), caractérisée en ce que ladite composition est un latex inverse comprenant de 20 % à 70% en poids, et de préférence de 25 % à 45 % en poids, d'un polyélectrolyte anionique, branché ou réticulé, à base d'un monomère possédant une fonction acide fort, caractérisé en ce que :
a) l'on émulsionne une solution aqueuse contenant les monomères et les éventuels additifs, dans une phase huile en présence d'un ou plusieurs agents émulsifiants de type eau dans huile,
b) l'on amorce la réaction de polymérisation par introduction dans l'émulsion formée en a), d'un initiateur de radicaux libres puis on la laisse se dérouler,
c) lorsque la réaction de polymérisation est terminée, l'on introduit un ou plusieurs agents émulsifiants de type huile dans eau à une température inférieure à 50°C.

Par « agent émulsifiant du type eau dans huile », on désigne des agents émulsifiants possédant une valeur HLB suffisamment faible pour foumir des émulsions eau dans huile tels que les polymères tensioactifs commercialisés sous le nom de HYPERMER™ ou tels que les extraits de sorbitan, comme le monooléate de sorbitan commercialisé par la Société SEPPIC sous le nom de marque MONTANE 80™, ou l'isostéorate de sorbitan commercialisé par SEPPIC sous le nom de MONTANE 70™.

Par « agent émulsifiant du type huile dans eau », on désigne des agents émulsifiants possédant une valeur HLB suffisamment élevée pour foumir des émulsions huile dans eau, tels que les esters de sorbitan éthoxylés comme l'oléate de sorbitan éthoxylé avec 20 moles d'oxyde d'éthylène, l'huile de ricin éthoxylée à 40 moles d'oxyde d'éthylène, le laurate de sorbitan éthoxylé à 20 moles d'oxyde d'éthylène ou l'alcool laurique éthoxylé à 7 moles d'oxyde d'éthylène.

Par polymère branché, on désigne un polymère non linéaire qui possède des chaînes pendantes de manière à obtenir, lorsque ce polymère est mis en solution dans l'eau, fort état d'enchevêtrement conduisant à des viscosités à bas gradient très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La composition préparée par le procédé selon l'invention, peut comporter des motifs réticulés et/ou des motifs branchés.

L'invention a notamment pour objet un procédé tel que défini précédemment, caractérisée en ce que la polymérisation de ses monomères précurseurs, est effectuée à un pH inférieur à 4 et, plus particulièrement inférieur ou égal à 3,5.

La fonction acide fort du monomère en comportant est notamment la fonction acide sulfonique ou la fonction acide phosphonique, partiellement ou totalement salifiées, et de préférence ledit monomère est choisi parmi l'acide 2-méthyl 2-[(1-oxo 2-propènyl) amino] 1-propane-sulfonique partiellement ou totalement salifié sous forme de sel de sodium ou de sel d'ammonium.

L'invention a plus particulièrement pour objet un procédé tel que défini précédemment, caractérisée en ce que le polyélectolyte anionique est réticulé et/ou branché avec un composé diéthylènique ou polyéthylènique dans la proportion molaire exprimée par rapport aux monomères mis en oeuvre, de 0,005% à 1%, et de préférence de 0,01% à 0,1% et, de préférence celle pour laquelle l'agent de réticulation et/ou l'agent de ramification est choisi parmi le méthacrylate d'éthylèneglycol, le diallyloxyacétate de sodium, le diacrylate de diéthylèneglycol, le diallyl urée, le triméthylol propane-triacrylate ou le méthylène-bis-acrylamide.

Le latex selon l'invention contient généralement de 2,5% à 15% en poids, et de préférence de 4% à 9% en poids, d'agents émulsifiants, parmi lesquels de 20% à 50%, notamment de 25% à 40% du poids total des agents émulsifiants présents sont du type eau dans huile (E/H) et dans laquelle de 80% à 50%, notamment de 75% à 60%, du poids total des agents émulsifiants, sont du type huile dans eau (H/E). De tels latex sont aussi objet de la présente invention.

Selon un aspect particulier, la composition telle que définie précédemment, est caractérisée en ce que la phase huile représente de 15% à 40%, de préférence de 20% à 25%, de son poids total.

Cette phase huile est constituée soit par une huile minérale commerciale contenant des hydrocarbures saturés de type paraffinique, isoparaffinique, cycloparaffinique, présentant à température ambiante, une densité entre 0.7 et 0.9 et un point d'ébullition supérieur à 180°C, telle que par exemple l'EXXOL™ D 100 S ou le MARCOL ™52 commercialisé par EXXON CHEMICAL, l'isohexadécane ou l'isododécane commercialisé par BAYER, soit par une huile végétale, soit une huile de synthèse, soit par un mélange de plusieurs de ces huiles.

Selon un aspect préféré de la présente invention, la phase huile est constituée de MARCOL™52 ou d'isohexadécane ; l'isohexadécane, qui est identifié dans Chemical Abstracts par le numéro RN = 93685-80-4, est un mélange d'isoparaffines en C₁₂, C₁₆ et C₂₀ contenant au moins 97 % d'isoparaffines en C₁₆, parmi lesquelles le constituant principal est le 2,2,4,4,6,8,8-heptaméthyl nonane (RN = 4390-04-9). Il est commercialisé en France par la société BAYER. Le MARCOL™ 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993).

Les latex contiennent entre 20 % et 50 % d'eau. Les latex selon l'invention peuvent également contenir divers additifs tels que des agents complexants, des agents de transfert, ou des agents limiteurs de chaîne.

Selon un autre aspect de la présente invention, celle-ci a pour objet un procédé de préparation de la composition telle que définie précédemment, caractérisé en ce que :
a) l'on émulsionne une solution aqueuse contenant les monomères et les éventuels additifs, dans une phase huile en présence d'un ou plusieurs agents émulsifiants de type eau dans huile,
b) l'on amorce la réaction de polymérisation par introduction dans l'émulsion formée en a), d'un initiateur de radicaux libres puis on la laisse se dérouler,
c) lorsque la réaction de polymérisation est terminée, l'on introduit un ou plusieurs agents émulsifiants de type huile dans eau à une température inférieure à 50°C.

Selon une variante de ce procédé, le milieu réactionnel issu de l'étape b), est concentré par distillation, avant la mise en oeuvre de l'étape c).

Selon une variante préférée du procédé tel que défini précédemment, la réaction de polymérisation est amorcée par un couple oxydo-réducteur, qui peut être de nature organique ou minérale, tel que le couple hydroperoxyde de cumène - métabisulfite de sodium, ou le couple hydroperoxyde de eumène-chlorure thionyle (SOCl₂) à une température inférieure ou égale à 10°C, puis conduite soit de manière quasi-adiabatique jusqu'à une température supérieure ou égale à 40°C et, plus particulièrement supérieure ou égale à 50°C, soit en contrôlant l'évolution de la température. Selon une autre variante du procédé tel que défini précédemment, la réaction de polymérisation est réalisée à température constante. Dans ce cas, il est avantageux d'utiliser l'azobis (isobutyronitrile) (AIBN) entre 40°C et 45°C.

Selon une autre mise en oeuvre préférée du procédé, la solution aqueuse de départ est ajustée à un pH inférieur ou égal à 3,5.

L'invention a aussi pour objet l'utilisation de la composition telle que définie précédemment pour préparer une composition topique cosmétique, dermo-pharmaceutique ou pharmaceutique.

Une composition topique selon l'invention, destinée à être appliquée sur la peau ou les muqueuses de l'homme ou de l'animal, peut consister en une émulsion topique comprenant au moins une phase aqueuse et au moins une phase huile. Cette émulsion topique peut être du type huile dans eau. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau et des muqueuses. Dans ce dernier cas, la composition topique comporte alors un principe actif qui peut par exemple consister en un agent anti-inflammatoire, un myorelaxant, un antifongique ou un antibactérien.

Lorsque la composition topique est utilisée en tant que composition cosmétique destinée à être appliquée sur la peau ou les muqueuses, elle peut ou non comporter un principe actif, par exemple un agent hydratant, un agent bronzant, un filtre solaire, un antirides, un agent à visée amincissante, un agent antiradicalaire, un agent antiacnéique ou un antifongique.

Une composition topique selon l'invention comporte habituellement entre 0,1 % et 10 % en poids de l'agent épaississant défini ci-dessus. Le pH de la composition topique est de préférence supérieur ou égal à 5, plus préférentiellement, il est compris entre 6 et 12.

La composition topique peut en outre comporter des composés classiquement compris dans ce type de compositions, par exemple des parfums, des conservateurs, des colorants, des émollients ou des tensioactifs.

Selon encore un autre aspect, l'invention concerne l'utilisation du nouvel agent épaississant conforme à l'invention mentionné ci-dessus, pour épaissir et émulsionner une composition topique comprenant au moins une phase aqueuse

La composition selon l'invention est un substitut intéressant à celles vendues sous le nom SEPIGEL ® 305 ou SEPIGEL ® 501 par la demanderesse, car elle présente aussi une bonne compatibilité avec les autres excipients utilisés pour la préparation de formulations telles que les laits, les lotions, les crèmes, les savons, les bains, les baumes, les shampooings ou les après-shampooings

Elle est notamment compatible avec les concentrés décrits et revendiqués dans les publications internationales WO 92/06778, WO 95/04592, W095/13863, WO 96/37285, WO 98/22207, WO 98/47610 ou FR 2734 496, avec les agents tensioactifs décrits dans WO 93/08204.

Elle est particulièrement compatible avec le MONTANOV™ 68, le MONTANOV™ 82, le MONTANOV ™202, ou le SEPIPERL™ N. Elle peut également être utilisée dans des émulsions du type de celles décrites et revendiquées dans EP 0 629 396 et dans les dispersions aqueuses cosmétiquement ou physiologiquement acceptable avec un composé organo polysiloxane choisi, par exemple parmi ceux décrits dans WO 93/05762 ou dans WO 93/21316.

Elle peut également être utilisée pour former des gels aqueux à pH acide cosmétiquement ou physiologiquement acceptables, tels que ceux décrit dans WO 93/07856; elle peut également être utilisée en association avec des celluloses non-ioniques, pour former par exemple des gels de coiffage tels que ceux décrits dans EPO 684 024, ou encore en association avec des esters d'acides gras et de sucre , pour former des compositions pour le traitement du cheveux ou de la peau telles que celles décrites dans EP 0 603 019. ou encore dans les shampooings ou après-shampooings tels que décrits et revendiqués dans WO 92/21316 ou enfin en association avec un homopolymère anionique tels que le CARBOPOL™ pour former des produits de traitement des cheveux comme ceux décrits dans DE 195 23596.

La composition selon l'invention est également compatible avec les principe actifs tels que par exemple, les agents auto-bronzants comme le dihydroxy-acétone (DHA) ou les agents anti-acné; elle peut donc être introduite dans des compositions auto-bronzantes comme celles revendiquées dans EP 0 715 845, EP 0 604249, EP 0576188 ou dans WO 93/07902

Elle est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561, WO 98/09611, WO 99/00109.

Les exemples qui suivent ont pour but d'illustrer la présente invention sans toutefois la limiter.

### Exemple 1:

### 1) Préparation d'un latex inverse

a) On charge dans un bécher, sous agitation
   - 220 g d'eau permutée
   - 138,1 g d'une solution aqueuse d'hydroxyde de sodium à 48 % (en poids)
   - 343,5 g de l'acide 2-methyl-2[(1-oxo-2 propenyl) amino] 1-propane sulfonique
   - 0,18 g de diéthylène triamine pentacétate de sodium
   - 0,22 g de méthylène-bis-acrylamide
le pH de la phase aqueuse précédemment décrit est ajusté à 3,5 et la quantité de phase aqueuse est complétée jusqu'à concurrence de 707 g par ajout d'eau permutée.

Parallèlement, on prépare une phase organique en introduisant dans un bécher agité successivement :
- 220 g d'isohexadécane
- 22 g de Montane 80 VG (oléate de sorbitan commercialisé par SEPPIC)
- 0,2 g azo-bis-isobutyronitrile

La phase aqueuse est introduite progressivement dans la phase organique puis soumise à une agitation mécanique violente de type ultra-turrax® commercialisé par IKA.

L'émulsion obtenue est alors transférée dans un réacteur de polymérisation. L'émulsion est soumise à un barbotage d'azote important de manière à éliminer l'oxygène et refroidit à environ 5-6°C.

On introduit alors 5ml d'une solution contenant 0,42 % (en poids) d'hydroperoxyde de cumène dans l'isohexadécane.

Après un temps suffisant pour une bonne homogénéisation de la solution, on introduit alors une solution aqueuse de métabisulfite de sodium (0,4 g dans 100 ml d'eau) à raison de 0,5 ml/minute. L'introduction est réalisée pendant environ 60 minutes.

Pendant cette introduction, on laisse monter la température dans le réacteur de polymérisation jusqu'à la température finale de polymérisation.

On maintient alors le milieu réactionnel pendant environ 90 minutes à cette température.

L'ensemble est refroidi jusqu'à une température d'environ 35°C et on introduit lentement 30 g d'oléate de sorbitan éthoxylé à 20 moles d'oxyde d'éthylène.

On obtient l'émulsion désirée ayant les viscosités suivantes :

Viscosité à 25°C du latex à 3 % dans l'eau (Brookfield RVT, Mobile 6, vitesse 5) : n = 93800 mPas.

### 2) Stabilité du latex au pH

Les mesures de viscosité de gels à 3 % du latex inverse préparé au paragraphe précédent en pH acide, ajusté par une solution diluée d'aide lactique, montrent que celui-ci est stable :

| pH | n |
|---|---|
| 3,04 | 76.800 |
| 4,00 | 94.000 |
| 5.00 | 93.000 |

Viscosité à 3 % dans l'eau (Brookfield RVT, Mobile 6, vitesse 5).

### 3) effet « break »

On prépare avec le latex préparé au paragraphe 1 précédent, des formules cosmétiques comprenant :
0,5%, 1%, 1,5%, 2%, 2,5% ou 3% de latex
5% de SIMULSOL 165,
20% de LANOL 1688
0,5% de SEPICIDE HB
eau qsp 100%

On constate que le toucher de ces émulsions est très particulier à partir de 1% de polymère dans la solution et que cette différence s'accentue avec l'augmentation de la concentration ; il s'agit d'un toucher très frais au début qui fond complètement sur la peau, toucher que l'on ne ressent pas du tout avec les latex d'état de la technique.

### Exemple 2 : Crème de soin

| | |
|---|---|
| Cyclométhicone | 10% |
| Composé de l'exemple 1 | 0,8 % |
| MONTANOV™68 | 4,5 % |
| Conservateur | 0,65 % |
| Lysine | 0,025 % |
| EDTA (sel disodique) | 0,05 % |
| Gomme de xanthane | 0,2 % |
| Glycérine | 3% |
| Eau | qsp 100 % |

### Exemple 3 : Crème de soin

| | |
|---|---|
| Cyclométhicone | 10 % |
| Composé de l'exemple 1 | 0,8 % |
| MONTANOV ™ 68 | 4,5 % |
| Perfluoropolymethylisopropylethe r | 0,5 % |
| Conservateur | 0,65 % |
| Lysine | 0,025 % |
| EDTA (sel disodique) | 0,05 % |
| PEMULEN™TR | 0,2 % |
| Glycérine | 3 % |
| Eau | qsp 100 % |

### Exemple 4 : Baume après-rasage

| FORMULE | | |
|---|---|---|
| A | Composé de l'exemple 1 | 1.5 % |
| | Eau | q.s.p 100 % |
| B | MICROPEARL™ M 100 | 5,0 % |
| | SEPICIDE™ CI | 0,50 % |
| | Parfum | 0,20 % |
| | Ethanol 95° | 10,0 % |

### MODE OPERATOIRE

Ajouter B dans A.

### Exemple 5 : Emulsion satinée pour le corps

| FORMULE | | |
|---|---|---|
| A | SIMULSOL™ 165 | 5,0 % |
| | LANOL™ 1688 | 8,50 % |
| | Beurre de Karité | 2 % |
| | Huile de paraffine | 6,5 % |
| | LANOL™ 14M | 3 % |
| | LANOL™ S | 0,6 % |
| B | Eau | 66,2 % |
| C | MICROPEARL™ M 100 | 5 % |
| D | Composé de l'exemple 1 | 3 % |
| E | SEPICIDE™ Cl | 0,3 % |
| | SEPICIDE™ HB | 0,5 % |
| | MONTEINE™ CA | 1 % |
| | Parfum | 0,20 % |
| | Acétate de vitamine E | 0,20 % |
| | Sodium pyrolidinone carboxylate | 1 % (agent hydratant) |

### MODE OPERATOIRE

Ajouter C dans B, émulsionner B dans A à 70°C, puis ajouter D à 60°C puis E à 30°C.

### Exemple 6 : Lait corporel

| FORMULE | | |
|---|---|---|
| A | SIMULSOL™ 165 | 5,0 % |
| | LANOL™ 1688 | 12,0 % |
| | LANOL™ 14M | 2,0 % |
| | Alcool cétylique | 0,3 % |
| | SCHERCEMOL™ OP | 3 % |
| B | Eau | q.s.p. 100% |
| C | Composé de l'exemple 1 | 0,35 % |
| D | SEPICIDE ™ CI | 0,2 % |
| | SEPICIDE ™ HB | 0,5 % |
| | Parfum | 0,20 % |
| (Le SCHERCEMOL ™ OP est un ester émollient à effet non gras) | | |

### MODE OPERATOIRE

Emulsionner B dans A vers 75°C; ajouter C vers 60°C, puis D vers 30°C

### Exemple 7 : crème H/E

| FORMULE | | |
|---|---|---|
| A | SIMULSOL™165 | 5,0% |
| | LANOL™1688 | 20,0% |
| 1,0% (additif à effet stabilisant | | |
| B | Eau | q.s.p. 100% |
| C | Composé de l'exemple 1 | 2,50% |
| D | SEPICIDE™ Cl | 0,20% |
| | SEPICIDE™ HB | 0,30% |

### MODE OPERATOIRE

Introduire B dans A vers 75°C ; ajouter C vers 60°C, puis D vers 45°C

### Exemple 8 : gel solaire non gras

| FORMULE | | |
|---|---|---|
| A | Composé de l'exemple 1 | 3,00% |
| | Eau | 30% |
| B | SEPICIDE™ C | 0,20% |
| | SEPICIDE™ HB | 0,30% |
| | Parfum | 0,10% |
| C | Colorant | q.s. |
| | Eau | 30% |
| D | MICROPEARL™ M 100 | 3,00% |
| | Eau | q.s.p 100% |
| E | Huile de silicone | 2,0% |
| | PARSOL™ MCX | 5,00% |

### MODE OPERATOIRE

Introduire B dans A; ajouter C,puis D, puis E.

### Exemple 9 : Lait solaire

| FORMULE | | |
|---|---|---|
| A | SEPIPERL™N | 3,0% |
| | Huile de sésame | 5,0% |
| | PARSOL™ MCX | 5,0% |
| | Carraghénane λ | 0,10% |
| B | Eau | q.s.p.100% |
| C | Composé de l'exemple 1 | 0,80% |
| D | Parfum | q.s. |
| | Conservateur | q.s. |

### MODE OPERATOIRE

Emulsionner B dans A à 75°C puis ajouter C vers 60°C, puis D vers 30°C et ajuster le pH si nécessaire

### Exemple 10 : Gel de massage

| FORMULE | | |
|---|---|---|
| A | Composé de l'exemple 1 | 3,5% |
| | Eau | 20,0% |
| B | Colorant | 2 gouttes/100g |
| | Eau | q.s. |
| C | Alcool | 10% |
| | Menthol | 0,10% |
| D | Huile de silicone | 5,0% |

Ajouter B dans A; puis ajouter au mélange, C puis D

### MODE OPERATOIRE

### Exemple 11 : gel soin de massage

| FORMULE | | |
|---|---|---|
| A | Composé de l'exemple 1 | 3,00% |
| | Eau | 30% |
| B | SEPICIDE™ Cl | 0,20% |
| | SEPICIDE™ HB | 0,30% |
| | Parfum | 0,05% |
| C | colorant | q.s. |
| | Eau | q.s.p 100% |
| D | MICROPEARL™ SQL | 5,0% |
| | LANOL™ 1688 | 2% |

### MODE OPERATOIRE

Préparer A; additionner B, puis C, puis D.

### Exemple 12 : Gel coup d'éclat

| FORMULE | | |
|---|---|---|
| A | Composé de l'exemple 1 | 4% |
| | Eau | 30% |
| B | ELASTINE HPM | 5,0% |
| C | MICROPEARL™ M 100 | 3% |
| | Eau | 5% |
| D | SEPICIDE™ CI | 0,2% |
| | SEPICIDE™ HB | 0,3% |
| | Parfum | 0,06% |
| | Sodium pyrolidinone carboxylate 50% | 1% |
| | Eau | q.s.p. 100% |

### MODE OPERATOIRE

Préparer A; additionner B, puis C, puis D.

### Exemple 13 : Lait corporel

| FORMULE | | |
|---|---|---|
| A | SEPIPERL™N | 3,0% |
| | Triheptonate de glycerol | 10,0% |
| B | Eau | q.s.p. 100% |
| C | Composé de l'exemple 1 | 1,0% |
| D | Parfum | q.s. |
| | Conservateur | q.s. |

### MODE OPERATOIRE

Fondre A à environ 75°C. Emulsionner B dans A à 75°C puis ajouter C vers 60°C, puis D.

### Exemple 14 : Emulsion démaquillante à l'huile d'amande douce

| FORMULE | |
|---|---|
| MONTANOV™68 | 5% |
| Huile d'amandes douces | 5% |
| Eau | q.s.p. 100% |
| Composé de l'exemple 1 | 0,3% |
| Glycérine | 5% |
| Conservateur | 0,2% |
| Parfum | 03% |

### Exemple 15 : Crème hydratante pour peaux grasses

| FORMULE | |
|---|---|
| MONTANOV™ 68 | 5% |
| Cétylstéaryloctanoate | 8% |
| Octyl palmitate | 2% |
| Eau | q.s.p. 100% |
| Composé de l'exemple 1 | 0,6% |
| MICROPEARL™ M100 | 3,0% |
| Mucopolysaccharides | 5% |
| SEPICIDE™ HB | 0,8 |
| Parfum | 03% |

### Exemple 16 : Baume après-rasage apaisant sans alcool

| FORMULE | |
|---|---|
| Mélange de lauryl aminoacides | 0,1% à 5% |
| Aspartate de magnésium et de potassium | 0,002% à 0,5% |
| LANOL™99 | 2% |
| Huile d'amandes douces | 0,5% |
| Eau | q.s.p.100% |
| Composé de l'exemple 1 | 3% |
| SEPICIDE™ HB | 0,3% |
| SEPICIDE™ Cl | 0,2% |
| Parfum | 0,4% |

### Exemple 17 : Crème aux AHA pour peaux sensibles

| FORMULE | |
|---|---|
| Mélange de lauryl aminoacides | 0,1 % à 5% |
| Aspartate de magnésium et de potassium | 0,002% à 0,5% |
| LANOL™ 99 | 2% |
| MONTANOV™ 68 | 5,0% |
| Eau | q.s.p.100% |
| Composé de l'exemple 1 | 1,50% |
| Acide gluconique | 1,50% |
| Triéthanolamine | 0,9% |
| SEPICIDE™ HB | 0,3% |
| SEPICIDE™Cl | 0,2% |
| Parfum | 0,4% |

### Exemple 18 : Soin apaisant après-soleil

| FORMULE | |
|---|---|
| Mélange de lauryl aminoacides | 0,1% à 5% |
| Aspartate de magnésium et de potassium | 0,002% à 0,5% |
| LANOL™ 99 | 10,0% |
| Eau | q.s.p.100% |
| Composé de l'exemple 1 | 2,50% |
| SEPICIDE™ HB | 0,3% |
| SEPICIDE™ Cl | 0,2% |
| Parfum | 0,4% |
| Colorant | 0,03% |

### Exemple 19 : Lait démaquillant

| FORMULE | |
|---|---|
| SEPIPERL™N | 3% |
| PRIMOL 352 | 8,0% |
| Huile d'amandes douces | 2% |
| Eau | q.s.p.100% |
| Composé de l'exemple 1 | 0,8% |
| Conservateur | 0,2% |

### Exemple 20 : Lait corporel

| FORMULE | |
|---|---|
| SEPIPERL™N | 3,5% |
| LANOL™ 37T | 8,0% |
| SOLAGUM™L | 0,05% |
| Eau | q.s.p.100% |
| Benzophénone | 2,0% |
| Diméthicone 350cPs | 0,05% |
| Composé de l'exemple 1 | 0,8% |
| Conservateur | 0,2% |
| Parfum | 0,4% |

### Exemple 21: émulsion fluide à pH alcalin

| | |
|---|---|
| MARCOL™ 82 | 5,0% |
| NaOH | 10,0% |
| Eau | q.s.p.100% |
| Composé de l'exemple 1 | 1,5% |

### Exemple 22 : Fond de teint fluide

| FORMULE | |
|---|---|
| SIMULSOL™ 165 | 5,0% |
| LANOL™ 84D | 8,0% |
| LANOL™99 | 5,0% |
| Eau | q.s.p.100% |
| Pigments et charges minérales | 10,0% |
| Composé de l'exemple 1 | 1,2% |
| Conservateur | 0,2% |
| Parfum | 0,4% |

### Exemple 23 : Lait solaire

| FORMULE | |
|---|---|
| SEPIPERL™N | 3,5% |
| LANOL™ 37T | 10.0% |
| PARSOL NOX™ | 5,0% |
| EUSOLEX™ 4360 | 2,0% |
| Eau | q.s.p. 100% |
| Composé de l'exemple 1 | 1,8% |
| Conservateur | 0,2% |
| Parfum | 0,4% |

### Exemple 24 : Gel contour.des yeux

| FORMULE | |
|---|---|
| Composé de l'exemple 1 | 2,0% |
| Parfum | 0,06% |
| Sodium pyrrolidinonecarboxylate | 0,2% |
| DOW CORNING™ 245 FLuid | 2,0% |
| Eau | q.s.p. 100% |

### Exemple 25: composition de soin non rincée

| FORMULE | |
|---|---|
| Composé de l'exemple 1 | 1,5% |
| Parfum | q.s |
| Conservateur | q.s. |
| DOW CORNING™ X2 8360 | 5,0% |
| DOW CORNING™ Q2 1401 | 15,% |
| Eau | q.s.p. 100% |

### Exemple 26: gel amincissant

| | |
|---|---|
| Composé de l'exemple 1 | 5 % |
| Ethanol | 30 % |
| Menthol | 0,1 % |
| Caféine | 2,5 % |
| Extrait de ruscus | 2 % |
| Extrait de lierre | 2 % |
| SEPICIDE™HB | 1 % |
| Eau | q.s.p. 100 % |

### Exemple 27 : Baume après-rasage apaisant sans alcool

| FORMULE | | |
|---|---|---|
| A | LIPACIDE™ PVB | 1,0% |
| | LANOL™ 99 | 2,0% |
| | Huile d'amandes douces | 0,5% |
| B | Composé de l'exemple 1 | 3,5% |
| C | Eau | q.s.p. 100% |
| D | Parfum | 0,4% |
| | SEPICIDE™ HB | 0,4% |
| | SEPICIDE™ CI | 0,2% |

### Exemple 28: Gel rafraîchissant après-rasage

| FORMULE | | |
|---|---|---|
| A | LIPACIDE™ PVB | 0,5% |
| | LANOL™ 99 | 5,0% |
| | Composé de l'exemple 1 | 2,5% |
| B | eau | q.s.p.100% |
| C | MICROPEARL™ LM | 0,5% |
| | Parfum | 0,2% |
| | SEPICIDE™ HB | 0,3% |
| | SEPICIDE™ CI | 0,2% |

### Exemple 29: Soin pour les peaux grasses

| FORMULE | | |
|---|---|---|
| A | MICROPEARL™ M310 | 1,0% |
| | Composé de l'exemple 1 | 5,0% |
| | Isononanoate d'octyle | 4.0% |
| B | Eau | q.s.p.100% |
| C | SEPICONTROL™ A5 | 4,0% |
| | Parfum | 0.1% |
| | SEPICIDE™ HB | 0,3% |
| | SEPICIDE™ Cl | 0,2% |
| D | CAPIGEL™ 98 | 0,5% |
| | Eau | 10% |

### Exemple 30 : Crème aux AHA

| FORMULE | | |
|---|---|---|
| A | MONTANOV™ 68 | 5.0% |
| | LIPACIDE™ PVB | 1,05% |
| | LANOL™ 99 | 10.0% |
| B | Eau | q.s.p. 100% |
| | Acide gluconique | 1,5% |
| | TEA (triéthanolamine) | 0,9% |
| C | Composé de l'exemple 1 | 1,5% |
| D | Parfum | 0,4% |
| | SEPICIDE™ HB | 0,2% |
| | SEPICIDE™ Cl | 0,4% |

### Exemple 31 : Autobronzant non gras pour visage et corps

| FORMULE | | |
|---|---|---|
| A | LANOL™ 2681 | 3,0% |
| | Composé de l'exemple 1 | 2,5% |
| B | Eau | q.s.p.100% |
| | Dihydroxyacétone | 3,0% |
| C | Parfum | 0,2% |
| | SEPICIDE™ HB | 0,8% |
| | NaOH (hydroxyde de sodium) | qs pH = 5 |

### Exemple 32 : Lait solaire au monoï de Tahiti

| FORMULE | | |
|---|---|---|
| A | Monoï de Tahiti | 10% |
| | LIPACIDE™ PVB | 0,5% |
| | Composé de l'exemple 1 | 2,2% |
| B | Eau | q.s.p. 100% |
| C | Parfum | 0,1% |
| | SEPICIDE™ HB | 0,3% |
| | SEPICIDE™ CI | 0,1% |
| | Méthoxycinnamate d'octyle | 4,0% |

### Exemple 33 : Soin solaire pour le visage

| FORMULE | | |
|---|---|---|
| A | Cyclométhicone et diméthiconol | 4,0% |
| | Composé de l'exemple 1 | 3,5% |
| B | Eau | q.s.p. 100% |
| C | Parfum | 0,1% |
| | SEPICIDE™ HB | 0,3% |
| | SEPICIDE™ Cl | 0,21% |
| | Méthoxycinnamate d'octyle | 5,0% |
| | Micatitane | 2,0% |
| | Acide lactique | q.s.p. pH = 6,5 |

### Exemple 34 : Emulsion bronzante sans soleil

| FORMULE | | |
|---|---|---|
| A | LANOL™ 99 | 15% |
| | MONTANOV™ 68 | 5,0% |
| | Paraméthoxycinnamate d'octyle | 3,0% |
| B | Eau | q.s.p. 100% |
| | Dihydroxyacétone | 5,0% |
| | Phosphate monosodique | 0,2% |
| C | Composé de l'exemple 1 | 0,5% |
| D | Parfum | 0,3% |
| | SEPICIDE™ HB | 0,8% |
| | NaOH | q.s. pH=5. |

### Exemple 35 : Gel brillance

| | |
|---|---|
| Composé de l'exemple 1 | 1,5 % |
| Silicone volatil | 25 % |
| Monopropylèneglycol | 25 % |
| Eau déminéralisée | 10 % |
| Glycérine | qsp 100 % |

### Exemple 36 : Gel amincissant

| | |
|---|---|
| Composé de l'exemple 1 | 1,5 % |
| Isononylisononanoate | 2 % |
| Caféine | 5 % |
| Ethanol | 40 % |
| MICROPEARL™ LM | 2 % |
| Eau déminéralisée | qsp 100 % |
| Conservateur parfum | qs |

### Exemple 37 : Lait démaquillant

| | |
|---|---|
| SIMULSOL™ 165 | 4 % |
| MONTANOV™ 202 | 1 % |
| Caprylate-caprate triglyceride | 15 % |
| PECOSIL™ DCT | 1 % |
| Eau déminéralisée | qs |
| CAPIGEL™ 98 | 0,5 % |
| Composé de l'exemple 1 | 1 % |
| PROTEOL™ OAT | 2 % |
| NaOH | qsp pH 7 |

### Exemple 38 : Crème solaire

| | |
|---|---|
| SIMULSOL™ 165 | 3 % |
| MONTANOV™ 202 | 2 % |
| Benzoate C12-C15 | 8 % |
| PECOSIL™ PS 100 | 2 % |
| Diméthicone | 2 % |
| Cyclométhicone | 5 % |
| Octyl-méthoxy-cinnamate | 6 % |
| Benzophénone-3 | 4 % |
| Oxyde de Titane | 8 % |
| Gomme xanthane | 0,2 % |
| Butyléne-glycol | 5 % |
| Eau déminéralisée | qsp 100 % |
| Composé de l'exemple 1 | 1,5% |
| Conservateur, parfum | qs |

### Exemple 39 : Gel de soin peaux mixtes

| | |
|---|---|
| Composé de l'exemple 1 | 4 % |
| Squalane végétal | 5 % |
| Dimethicone | 1,5% |
| SEPICONTROL™ A5 | 4 % |
| Gomme xanthane | 0,3 % |
| Eau | qsp 100 % |
| Conservateur, Parfum | qs |

### Exemple 40 : Voile parfumé pour le corps

| | |
|---|---|
| Composé de l'exemple 1 | 1,5 % |
| Cyclométhicone | 5 % |
| Parfum | 2 % |
| MICROPEARL™ M100 | 5 % |
| Glycérine | 5 % |
| Eau déminéralisée | qsp 100 % |

### Exemple 41 : Crème vitaminée

| | |
|---|---|
| SIMULSOL™ 165 | 5 % |
| MONTANOV™ 202 | 1 % |
| Caprylic/capric triglycerides | 20 % |
| Palmitate de vitamine A | 0,2 % |
| Acetate de vitamine E | 1 % |
| MICROPEARL™ M305 | 1,5 % |
| Composé de l'exemple 1 | 0,7 % |
| Eau | qsp 100 % |
| Conservateur, parfum | qs |

Le MONTANOV™ 68 (cétéaryl glucoside), est une composition auto-émulsionnable telle que décrite dans WO 92/06778, commercialisée par la société SEPPIC.

Le MICROPEARL™ M 100 est une poudre ultra fine au toucher très doux et à action matifiante commercialisée par la société MATSUMO

Le SEPICIDE™ Cl, imidazolidine urée, est un agent conservateur commercialisé par la société SEPPIC.

PEMULEN™ TR est un polymère acrylique commercialisé par GOODRICH.

Le SIMULSOL™ 165 est du stéarate de glycérol auto-émulsionnable commercialisée par la société SEPPIC.

Le LANOL™ 1688 est un ester émollient à effet non gras commercialisé par la société SEPPIC.

Le LANOL™ 14M et le LANOL ® S sont des facteurs de consistance commercialisés par la société SEPPIC.

Le SEPICIDE™ HB , qui est un mélange de phénoxyéthanol, de méthyl paraben, d'éthylparaben, de propylparaben et de butylparaben, est un agent conservateur commercialisé par la société SEPPIC.

La MONTEINE™ CA est un agent hydratant commercialisé par la société SEPPIC.

Le SCHERCEMOL™ OP est un ester émollient à effet non gras.

Le LANOL™ P est un additif à effet stabilisant commercialisé par la société SEPPIC.

Le PARSOL™ MCX est de l'octyl paraméthoxycinnamate; commercialisé par la société GIVAUDAN.

Le SEPIPERL™ N est un agent nacrant, commercialisé par la société SEPPIC, à base d'un mélange d'alkyl poly glucosides tels que ceux décrits dans WO 95/13863.

Le MICROPEARL™ SQL est un mélange de micro particules renfermant du squalane qui se libère sous l'action du massage; il est commercialisé par la société MATSUMO.

Le LANOL™ 99 est de l'isononyl isononanoate commercialisé par la société SEPPIC.

Le LANOL™ 37T est du triheptanoate de glycérol, commercialisé par la société SEPPIC.

Le SOLAGUM™ L est un carraghénane commercialisé par la société SEPPIC.

Le MARCOL™ 82 est une huile de paraffine commercialisée par la société EXXON.

Le LANOL™ 84D est du malate de dioctyle commercialisé par la société SEPPIC.

Le PARSOL NOX™ est un filtre solaire commercialisé par la société GIVAUDAN.

l'EUSOLEX™ 4360 est un filtre solaire commercialisé par la société MERCK.

Le DOW CORNING™ 245 Fluid est de la cyclométhicone, commercialisée par la société DOW CORNING.

Le LIPACIDE™ PVB, est un hydrolysat de protéines de blé acylé commercialisé par la société SEPPIC.

Le MICROPEARL™ LM est un mélange de squalane, de polyméthylméthacrylate et de menthol, commercialisé par la société SEPPIC.

Le SEPICONTROL™ A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998.

Le CAPIGEL™ 98 est un copolymère acrylique commercialisé par la société SEPPIC.

Le LANOL™ 2681 est un mélange caprylate, caprate de coprah, commercialisé par la société SEPPIC.

Le MONTANOV™ 202, est une composition APG/alcools gras telle que décrite dans WO9 98/47610, commercialisée par la société SEPPIC.

## Revendications

1. Procédé de préparation d'une composition sous forme d'un latex inverse, comprenant une phase huile, une phase aqueuse, au moins un agent émulsifiant de type eau dans huile (E/H), au moins un agent émulsifiant de type huile dans eau (H/E), de 20 % à 70 % en poids, et de préférence de 25 % à 45 % en poids, d'un polyélectrolyte anionique, branché ou réticulé, à base d'un monomère possédant une fonction acide fort **caractérisé en ce que** :
a) l'on émulsionne une solution aqueuse contenant le monomère et les éventuels additifs préalablement ajustée à un pH inférieur à 4, dans une phase huile en présence d'un ou plusieurs agents émulsifiants de type eau dans huile,
b) l'on amorce la réaction de polymérisation par introduction dans l'émulsion formée en a), d'un initiateur de radicaux libres puis on la laisse se dérouler,
c) lorsque la réaction de polymérisation est terminée, l'on introduit un ou plusieurs agents émulsifiants de type huile dans eau à une température inférieure à 50°C
et **caractérisé en ce que** l'on opère en l'absence de monoacrylamide.

2. Procédé tel que défini à la revendication 1, **caractérisé en ce que** la solution aqueuse de départ est préalablement ajustée à un pH inférieur ou égal à 3,5.

3. Procédé tel que défini à l'une des revendications 1 ou 2, **caractérisé en ce que** la fonction acide fort du monomère précurseur du polyélectrolyte anionique est la fonction acide sutfonique ou la fonction acide phosphonique, et de préférence ledit monomère est l'acide 2-méthyl 2-[(1-oxo 2-propènyi) amino] 1-propane-sulfonique.

4. Procédé tel que défini à l'une quelconque des revendications 1 à 3, **caractérisé en ce que** l'un des additifs présents dans la solution aqueuse de l'étape a) est un agent de réticulation et/ou un agent de ramification choisi parmi les composés diéthylèniques ou polyéthylèniques dans la proportion molaire exprimée par rapport aux monomères mis en oeuvre, de 0,005 % à 1 %, et de préférence de 0,01 % à 0,1 %.

5. Procédé tel que défini à la revendication 4, dans lequel l'agent de réticulation et/ou l'agent de ramification est choisi parmi le méthacrylate d'éthylèneglycol, le diallyloxyacétate de sodium, le diacrylate d'éthylèneglycol, le diallyl urée, le triméthylol propane-triacrylate ou le méthylène-bis-acrylamide.

6. Variante du procédé tel que défini à l'une des revendications 1 à 5, selon laquelle le milieu réactionnel issu de l'étape b), est concentré par distillation, avant la mise en oeuvre de l'étape c).

7. Composition comprenant une phase huile, une phase aqueuse, au moins un agent émulsifiant de type eau dans huile (E/H), au moins un agent émulsifiant de type huile dans eau (H/E), **caractérisée en ce que** ladite composition est un latex inverse comprenant de 20 % à 70 % en poids, et de préférence de 25 % à 45 % en poids, d'un polyélectrolyte anionique, branché ou réticulé, à base d'un monomère possédant une fonction acide fort, **caractérisée en ce que** ladite composition contient de 2,5 % à 15 % en poids, et de préférence de 4 % à 9 % en poids, d'agents émulsifiants parmi lesquels de 20 % à 50 %, notamment de 25 % à 40 % du poids total des agents émulsifiants présents sont du type eau dans huile (E/H) et parmi lesquels de 80% à 50%, notamment de 75 % à 60 %, du poids total des agents émulsifiants, sont du type huile dans eau (H/E) et **caractérisée en ce que** ladite composition est exempte de mono-acrylamide.

8. Composition telle que définie à la revendication 7, dans laquelle la fonction acide fort du monomère est la fonction acide sulfonique ou la fonction acide phosphonique, partiellement ou totalement salifiées, et de préférence ledit monomère est choisi parmi l'acide 2-méthyl 2-[(1-oxo 2-propényl) amino] 1-propane-sulfonique partiellement ou totalement salifié sous forme de sel de sodium ou de sel d'ammonium.

9. Composition telle que définie à l'une des revendications 7 ou 8, dans laquelle le polyélectrolyte anionique est réticulé et/ou branché avec des composés diéthylèniques ou polyéthylèniques dans la proportion molaire exprimée par rapport aux monomères mis en oeuvre, de 0,005 % à 1 %, et de préférence de 0,01 % à 0,1 %, ledit agent de réticulation et/ou l'agent de ramification étant de préférence choisi parmi le méthacrylate d'éthylèneglycol, le diallyloxyacétate de sodium, le diacrylate d'éthylèneglycol, le diallyl urée, le triméthylol propane-triacrylate ou le méthylène-bis-acrylamide.

10. Composition telle que définie à l'une des revendications 7 à 9, **caractérisée en ce que** la phase huile représente de 15 % à 40 % de préférence de 20 % à 25 %, de son poids total.

11. Composition telle que définie à la revendication 10 dans laquelle la phase huile est constituée d'isohexadécane ou d'huile blanche minérale.

12. Composition telle que définie à l'une quelconque des revendications 7 à 11, **caractérisée en ce qu'**elle contient en outre un ou plusieurs additifs choisis notamment parmi les agents complexants, les agents de transfert ou les agents limiteurs de chaînes.

13. Utilisation de la composition telle que définie à l'une des revendications 7 à 12, pour préparer une composition topique cosmétique, dermo-pharmaceutique ou pharmaceutique.

14. Composition cosmétique, dermo-pharmaceutique ou pharmaceutique comprenant de 0,1 % à 10 % en poids d'un latex inverse tel que défini à l'une des revendications 7 à 12.

15. Composition telle que définie à la revendication 14, sous la forme d'un lait, d'une lotion, d'un gel, d'une crème, d'un savon, d'un bain moussant, d'un baume, d'un shampooing ou d'un après shampooing.

16. Composition apaisante pour peau sensible comprenant un latex inverse tel que défini à l'une des revendications 7 à 12 et un ou plusieurs aminoacides N-acylés.

## Claims

1. Process for the preparation of a composition in the form of an inverse latex, comprising an oil phase, an aqueous phase, at least one emulsifying agent of water-in-oil (W/O) type, at least one emulsifying agent of oil-in-water (O/W) type, and from 20% to 70% by weight, and preferably from 25% to 45% by weight, of a branched or crosslinked anionic polyelectrolyte based on a monomer having a strong acid functional group, **characterized in that**:
a) an aqueous solution comprising the monomer and the optional additives, adjusted beforehand to a pH of less than 4, is emulsified in an oil phase in the presence of one or more emulsifying agents of water-in-oil type,
b) the polymerization reaction is initiated by introduction into the emulsion formed in a) of a free radical initiator and then the polymerization reaction is allowed to take place,
c) when the polymerization reaction is completed, one or more emulsifying agents of oil-in-water type is/are introduced at a temperature of less than 50°C,
and **characterized in that** the process is carried out in the absence of monoacrylamide.

2. Process as defined in Claim 1, **characterized in that** the aqueous starting solution is adjusted beforehand to a pH of less than or equal to 3.5.

3. Process as defined in either of Claims 1 and 2, **characterized in that** the strong acid functional group of the precursor monomer of the anionic polyelectrolyte is the sulphonic acid functional group or the phosphonic acid functional group and preferably the said monomer is 2-methyl-2-[(1-oxo-2-propenyl)amino]-1-propanesulphonic acid.

4. Process as defined in any one of Claims 1 to 3, **characterized in that** one of the additives present in the aqueous solution of stage a) is a crosslinking agent and/or a branching agent chosen from diethylene or polyethylene compounds in the molar proportion, expressed with respect to the monomers employed, of 0.005% to 1% and preferably of 0.01% to 0.1%.

5. Process as defined in Claim 4, in which the crosslinking agent and/or the branching agent is chosen from ethylene glycol methacrylate, sodium diallyloxyacetate, ethylene glycol diacrylate, diallylurea, trimethylolpropane triacrylate or methylenebisacrylamide.

6. Alternative form of the process as defined in one of Claims 1 to 5, in which the reaction medium resulting from stage b) is concentrated by distillation before carrying out stage c).

7. Composition comprising an oil phase, an aqueous phase, at least one emulsifying agent of water-in-oil (W/O) type and at least one emulsifying agent of oil-in-water (O/W) type, **characterized in that** the said composition is an inverse latex comprising from 20% to 70% by weight, and preferably from 25% to 45% by weight, of a branched or crosslinked anionic polyelectrolyte based on a monomer having a strong acid functional group, **characterized in that** the said composition comprises from 2.5% to 15% by weight, and preferably from 4% to 9% by weight, of emulsifying agents, among which from 20% to 50%, in particular from 25% to 40%, of the total weight of the emulsifying agents present are of the water-in-oil (W/O) type and among which from 80% to 50%, in particular from 75% to 60%, of the total weight of the emulsifying agents are of the oil-in-water (O/W) type, and **characterized in that** the said composition is devoid of monoacrylamide.

8. Composition as defined in Claim 7, in which the strong acid functional group of the monomer is the partially or completely salified sulphonic acid functional group or the partially or completely salified phosphonic acid functional group and preferably the said monomer is chosen from 2-methyl-2-[(1-oxo-2-propenyl)amino]-1-propanesulphonic acid partially or completely salified in the form of the sodium salt or of the ammonium salt.

9. Composition as defined in either of Claims 7 and 8, in which the anionic polyelectrolyte is crosslinked and/or branched with diethylene or polyethylene compounds in the molar proportion, expressed with respect to the monomers employed, of 0.005% to 1%, and preferably of 0.01% to 0.1%, the said crosslinking agent and/or the branching agent preferably being chosen from ethylene glycol methacrylate, sodium diallyloxyacetate, ethylene glycol diacrylate, diallylurea, trimethylolpropane triacrylate or methylenebisacrylamide.

10. Composition as defined in one of Claims 7 to 9, **characterized in that** the oil phase represents from 15% to 40%, preferably from 20% to 25%, of its total weight.

11. Composition as defined in Claim 10, in which the oil phase is composed of isohexadecane or of white mineral oil.

12. Composition as defined in any one of Claims 7 to 11, **characterized in that** it additionally comprises one or more additives chosen in particular from complexing agents, transfer agents or chain-limiting agents.

13. Use of the composition as defined in one of Claims 7 to 12 for preparing a cosmetic, dermopharmaceutical or pharmaceutical topical composition.

14. Cosmetic, dermopharmaceutical or pharmaceutical composition comprising from 0.1% to 10% by weight of an inverse latex as defined in one of Claims 7 to 12.

15. Composition as defined in Claim 14, in the form of a milk, of a lotion, of a gel, of a cream, of a soap, of a foam bath, of a balm, of a shampoo or of a conditioner.

16. Soothing composition for sensitive skin, comprising an inverse latex as defined in one of Claims 7 to 12 and one or more N-acylated.amino acids.

## Patentansprüche

1. Verfahren zur Herstellung einer Zusammensetzung in Form eines inversen Latex, der eine Ölphase, eine wäßrige Phase, mindestens einen Emulgator des Wasser-in-Öl (W/O)-Typs, mindestens einen Emulgator des Öl-in-Wasser (O/W)-Typs, 20 Gew.-% bis 70 Gew.-%, vorzugsweise 25 Gew.-% bis 45 Gew.-%, eines verzweigten oder vernetzten anionischen Polyelektrolyten auf Basis eines Monomers mit starker Säurefunktion enthält, **dadurch gekennzeichnet, daß** man
a) eine wäßrige Lösung, die das Monomer und die gegebenenfalls vorhandenen Zusatzstoffe enthält und die zuvor auf einen pH-Wert von unter 4 eingestellt wurde, in einer Ölphase in Gegenwart von einem oder mehreren Emulgatoren des Wasser-in-Öl-Typs emulgiert,
b) die Polymerisationsreaktion durch Einbringen eines Radikalstarters in die unter a) gebildete Emulsion startet und dann ablaufen läßt,
c) nach Beendigung der Polymerisationsreaktion einen oder mehrere Emulgatoren des Öl-in-Wasser-Typs bei einer Temperatur von unter 50°C einbringt,
**dadurch gekennzeichnet, daß** man in Abwesenheit von Monoacrylamid arbeitet.

2. Verfahren nach Anspruch 1, **dadurch gekennzeichnet, daß** die wäßrige Ausgangslösung zuvor auf einen pH-Wert von 3,5 oder darunter eingestellt wird.

3. Verfahren nach einem der Ansprüche 1 oder 2, **dadurch gekennzeichnet, daß** es sich bei der starken Säurefunktion des als Vorstufe des anionischen Polyelektrolyten dienenden Monomers um die Sulfonsäurefunktion oder die Phosphonsäurefunktion handelt und es sich bei dem Monomer um 2-Methyl-2-[(1-oxo-2-propenyl)amino]-1-propansulfonsäure handelt.

4. Verfahren nach einem der Ansprüche 1 bis 3, **dadurch gekennzeichnet, daß** es sich bei einem der Zusatzstoffe, die in der wäßrigen Lösung von Schritt a) vorliegen, um ein Vernetzungsmittel und/oder ein Verzweigungsmittel aus der Gruppe der Diethylen- oder Polyethylenverbindungen in einem Molverhältnis von 0,005% bis 1%, vorzugsweise 0,01% bis 0,1% in bezug auf die verwendeten Monomere handelt.

5. Verfahren nach Anspruch 4, wobei das Vernetzungsmittel und/oder Verzweigungsmittel aus der Gruppe Ethylenglykolmethacrylat, Natriumdiallyloxyacetat, Ethylenglykoldiacrylat, Diallylharnstoff, Trimethylolpropantriacrylat oder Methylenbisacrylamid stammt bzw. stammen.

6. Variante des Verfahrens nach einem der Ansprüche 1 bis 5, wobei der in Schritt b) entstandene Ansatz vor Verwendung in Schritt c) durch Destillation eingeengt wird.

7. Zusammensetzung, die eine Ölphase, eine wäßrige Phase, mindestens einen Emulgator des Wasser-in-Öl (W/O)-Typs, mindestens einen Emulgator des Öl-in-Wasser (O/W)-Typs, **dadurch gekennzeichnet, daß** es sich bei dieser Zusammensetzung um einen inversen Latex handelt, der 20 Gew.-% bis 70 Gew.-%, vorzugsweise 25 Gew.-% bis 45 Gew.-%, eines verzweigten oder vernetzten anionischen Polyelektrolyten auf Basis eines Monomers mit starker Säurefunktion enthält, **dadurch gekennzeichnet, daß** diese Zusammensetzung 2,5 Gew.-% bis 15 Gew.-%, vorzugsweise 4 Gew.-% bis 9 Gew.-%, Emulgatoren enthält, von denen 20% bis 50%, insbesondere 25% bis 40% des Gesamtgewichts der vorhandenen Emulgatoren den Wasser-in-Öl (W/O)-Typ aufweisen und von denen 80% bis 50%, insbesondere 75% bis 60% des Gesamtgewichts der Emulgatoren den Öl-in-Wasser (O/W)-Typ aufweisen, **dadurch gekennzeichnet, daß** diese Zusammensetzung monoacrylamidfrei ist.

8. Zusammensetzung nach Anspruch 7, wobei es sich bei der starken Säurefunktion des Monomers um die Sulfonsäurefunktion oder Phosphonsäurefunktion, mit denen teilweise oder ganz ein Salz gebildet wurde, handelt, und das Monomer vorzugsweise aus der Gruppe 2-Methyl-2-[(1-oxo-2-propenyl)amino]-1-propansulfonsäure, mit der teilweise oder ganz ein Natrium- oder Ammoniumsalz gebildet wurde.

9. Zusammensetzung nach Anspruch 7 oder 8, in der der anionische Polyelektrolyt mit Diethylen- oder Polyethylenverbindungen in einem Molverhältnis von 0,005% bis 1%, vorzugsweise 0,01% bis 0,1%, in bezug auf die verwendeten Monomere vernetzt und/oder verzweigt ist, wobei das Vernetzungsmittel und/oder Verzweigungsmittel aus der Gruppe Ethylenglykolmethacrylat, Natriumdiallyloxyacetat, Ethylenglykoldiacrylat, Diallylharnstoff, Trimethylolpropantriacrylat oder Methylenbisacrylamid stammt bzw. stammen.

10. Zusammensetzung nach einem der Ansprüche 7 bis 9, **dadurch gekennzeichnet, daß** die Ölphase 15% bis 40%, vorzugsweise 20% bis 25%, ihres Gesamtgewichts ausmacht.

11. Zusammensetzung nach Anspruch 10, wobei die Ölphase aus Isohexadecan oder weißem Mineralöl besteht.

12. Zusammensetzung nach einem der Ansprüche 7 bis 11, **dadurch gekennzeichnet, daß** sie weiterhin ein oder mehrere Zusatzstoffe, insbesondere aus der Gruppe der Komplexbildner, der Kettenübertragungsreagentien und der Kettenabbruchmittel, enthalten.

13. Verwendung der Zusammensetzung nach einem der Ansprüche 7 bis 12 zur Herstellung einer topischen Kosmetikzusammensetzung, eines topischen Dermatotherapeutikums oder eines topischen Arzneimittels.

14. Kosmetikzusammensetzung, Dermatotherapeutikum oder Arzneimittel, die bzw. das 0,1 Gew.-% bis 10 Gew.-% eines inversen Latex nach einem der Ansprüche 7 bis 12 enthält.

15. Zusammensetzung nach Anspruch 14 in Form einer Milch, Lotion, Creme oder Seife bzw. eines Gels, Schaumbads, Balsams, Haarshampoos oder eines Haarmittels, das nach dem Shampoonieren verwendet wird.

16. Beruhigende Zusammensetzung für empfindliche Haut, die einen inversen Latex nach einem der Ansprüche 7 bis 12 und eine oder mehrere N-Acylaminosäuren enthält.
